# FASCICULE DE BREVET EUROPEEN

(11) **EP 0 663 814 B1**
(45) Date de publication et mention de la délivrance du brevet: **28.04.1999**
(21) Numéro de dépôt: 93901803.2
(22) Date de dépôt: 01.12.1992
(51) Int. Cl.: A61K 7/13

(54) **PROCEDE DE TEINTURE DES FIBRES KERATINIQUES AVEC UNE ALCOXYMETAPHENYLENEDIAMINE A pH ACIDE ET COMPOSITIONS MISES EN UVRE**
VERFAHREN ZUM FÄRBEN VON KERATINFASERN UNTER VERWENDUNG EINES ALKOXYMETAPHENYLENDIAMINS MIT SAUREM PH-WERT UND ZUSAMMENSETZUNG HIERFÜR
PROCESS FOR DYEING KERATIN FIBRES USING AN ACID PH ALCOXYMETEPHENYLENEDIAMINE AND COMPOSITIONS THEREFOR

(30) Priorité: 03.12.1991 FR 9114947
(43) Date de publication de la demande: 26.07.1995
(73) Titulaire: L'OREAL, 75008 Paris (FR)
(72) Inventeur: COTTERET, Jean, F-78480 Verneuil-sur-Seine (FR)
(74) Mandataire: Casalonga, Axel
(86) Numéro de dépôt international: FR9201115
(87) Numéro de publication internationale: WO9310744

(56) Documents cités:
- EP-A- 0 002 828
- EP-A- 0 029 964
- EP-A- 0 459 900
- WO-A-88/00042
- DE-A- 2 942 297
- DE-A- 3 007 997
- GB-A- 2 216 124

## Description

La présente invention est relative à un nouveau procédé de teinture des fibres kératiniques, en particulier des fibres kératiniques humaines telles que les cheveux, mettant en oeuvre une alcoxymétaphénylènediamine en association avec des bases d'oxydation et un agent oxydant en milieu acide et aux compositions mises en oeuvre au cours de ce procédé.

Il est connu de teindre les fibres kératiniques et en particulier les cheveux humains avec des compositions tinctoriales contenant en milieu alcalin, des précurseurs de colorants d'oxydation et en particulier des p-phénylènediamines, des ortho- ou para-aminophénols appelés généralement "bases d'oxydation".

On sait également que l'on peut faire varier les nuances obtenues avec ces bases d'oxydation en les associant à des coupleurs encore appelés modificateurs de coloration, choisis notamment parmi les métadiamines aromatiques, les méta-aminophénols et les méta-diphénols.

Ainsi, les demandes DE 2 942 297, EP 0 029 964 et WO 88 800 042 décrivent respectivement des dérivés de (2,4-diaminophénoxy) alkylamine, des dérivés de (2,4-diaminophénoxy) 2-hydroxypropyle 1-substitué et des dérivés 5-alcoxy 2,4-diaminoalkyl benzène comme coupleurs dans des procédés de teinture des cheveux mettant également en oeuvre des précurseurs de colorants d'oxydation en présence d'un agent oxydant. La demande de brevet GB 2 216 124 a trait, quant à elle, à un procédé de teinture des fibres kératiniques mettant en oeuvre des précurseurs de colorants d'oxydation avec des coupleurs bleus répondant à la formule 2,4-diamino 1-méthoxybenzène N-substitué en position 4 par un radical hydroxyalkyle, en présence d'un agent oxydant.

Par ailleurs, l'obtention de teintes chaudes est délicate dans le cas de la coloration d'oxydation à pH acide.

La demanderesse vient de découvrir que l'utilisation d'alcoxymétaphénylènediamines définies ci-après avec des bases d'oxydation en un mélange extemporané avec un oxydant, à un pH acide, permettait d'obtenir des teintures peu sélectives, c'est-à-dire que leur couleur est sensiblement la même sur cheveux naturels et sur cheveux sensibilisés, ayant une bonne résistance à la lumière, aux lavages, à la transpiration et aux intempéries.

La présente invention a donc pour objet un procédé de teinture des fibres kératiniques, en particulier des fibres kératiniques humaines telles que les cheveux, comprenant l'application sur ces fibres d'au moins une composition contenant une alcoxymétaphénylènediamine de formule (I), un précurseur de colorant d'oxydation encore appelé base d'oxydation et un agent oxydant, à pH acide.

L'invention a également pour objet un agent de teinture à deux composants, dont l'un des composants comprend l'alcoxymétaphénylènediamine de formule (I) ci-dessous, et le précurseur de colorant d'oxydation et l'autre l'agent oxydant à un pH acide, et en des quantités telles que le mélange présente un pH acide.

L'invention a également pour objet la composition prête à l'emploi, contenant les différents agents utilisés pour la teinture, en milieu acide, des cheveux.

D'autres objets de l'invention apparaîtront à la lecture de la description et des exemples qui suivent.

Le procédé de teinture des fibres kératiniques et en particulier des fibres kératiniques humaines telles que les cheveux, conforme à l'invention, est essentiellement caractérisé par le fait que l'on applique sur ces fibres une composition contenant dans un milieu approprié pour la teinture :
- au moins, comme coupleur, une alcoxymétaphénylènediamine de formule (I) suivante : 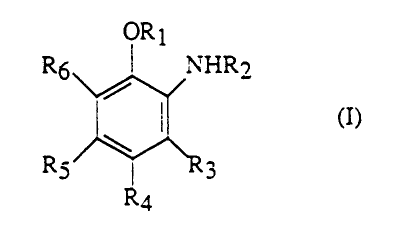 dans laquelle :
   R₁ représente un radical alkyle en C₁-C₄, mono- ou polyhydroxyalkyle en C₂-C₄, carboxyalkyle en C₁-C₄ ou aminoalkyle en C₁-C₄ dont le groupe amino peut être mono- ou disubstitué par un alkyle en C₁-C₄;
   R₂ représente un atome d'hydrogène, un radical alkyle en C₁-C₄, mono- ou polyhydroxyalkyle en C₂-C₄;
   R₃ et R₅, indépendamment l'un de l'autre, représentent H ou OR, R désignant un radical alkyle en C₁-C₄, mono- ou polyhydroxyalkyle en C₂-C₄;
   R₄ représente un atome d'hydrogène, un radical alkyle en C₁-C₄ ou NHR';
   R₆ représente un atome d'hydrogène, un radical alkyle en C₁-C₄, OR ou NHR', R ayant la signification indiquée ci-dessus;
   R' désigne un atome d'hydrogène, un radical alkyle en C₁-C₄, sous réserve que :
      a) un et un seul des radicaux R₄ et R₆ désigne NHR';
      b) R₃ et OR₁ ne peuvent désigner simultanément méthoxy lorsque R₂, R₅ et R₆ désignent simultanément l'hydrogène et R₄ le groupe NH₂;
      c) au moins un des radicaux R₃, R₄, R₅ ou R₆ désigne H;
      d) R₃, R₄ et R₅ ne peuvent désigner simultanément l'hydrogène lorsque R₆ désigne NH₂, R₂ désigne l'hydrogène et R₁ désigne méthyle;
      e) lorsque R₁ désigne éthyle et R₂, R₃, R₅ et R₆ désignent H, R₄ ne peut désigner NH₂;
      f) R₄ doit représenter NHR' lorsque R₁ désigne carboxyalkyle ou aminoalkyle;
         ainsi que les sels de ces composés;
- au moins un précurseur de colorant d'oxydation ou base d'oxydation;
- au moins un agent oxydant;
- le pH de la composition appliquée sur les fibres étant inférieur à 7.

La composition ne contient pas d'ions iodures en quantité suffisante pour oxyder l'alcoxymétaphénylènediamine de formule (I).

Les sels sont choisis parmi les sels d'addition d'acides, tels que d'acides chlorhydrique, bromhydrique, sulfurique, etc...

A titre de composés répondant à la formule (I), on peut citer :
- le 2,4-diaminoanisole,
- le 2-N-β-hydroxyéthylamino 4-amino 1,3,5-triméthoxybenzène,
- le 2-N-β-hydroxyéthylamino 4-amino 1-méthoxybenzène,
- le 2,4-diamino 1,5-diméthoxybenzène,
- le 2,4-diamino 5-méthoxyphénoxyéthanol,
- le 2,4-diamino 1,3,5-triméthoxybenzène,
- le 2,4-diamino 1,5-di-(β-hydroxyéthoxy)benzène,
- le 2,4-diaminophénoxyéthanol,
- le 2,4-diamino 6-méthyl 1-méthoxybenzène,
- le 2,4-diamino 1,6-diméthoxybenzène,
- le 2,6-diamino 4-méthyl 1-méthoxybenzène,
   ainsi que leurs sels.

Les précurseurs de colorants d'oxydation ou bases d'oxydation sont des composés connus qui ne sont pas des colorants en eux-mêmes et qui forment un colorant par un processus de condensation oxydative, soit sur eux-mêmes, soit en présence d'un coupleur ou modificateur. Ces composés comportent généralement un noyau aromatique portant des groupements fonctionnels, constitués : soit par deux groupements amino; soit par un groupement amino et un groupement hydroxy; ces groupements étant en position para ou ortho, l'un par rapport à l'autre.

Les précurseurs de colorants d'oxydation de type para, utilisés conformément à l'invention, sont choisis parmi les paraphénylènediamines, les para-aminophénols, les précurseurs hétérocycliques para, comme la 2,5-diaminopyridine, la 2-hydroxy 5-aminopyridine, la 2,4,5,6-tétra-aminopyrimidine.

Les para-aminophénols sont particulièrement intéressants pour l'obtention de nuances chaudes (rouges et cuivrées).

Parmi les paraphénylènediamines, on peut citer les composés répondant à la formule (II) : 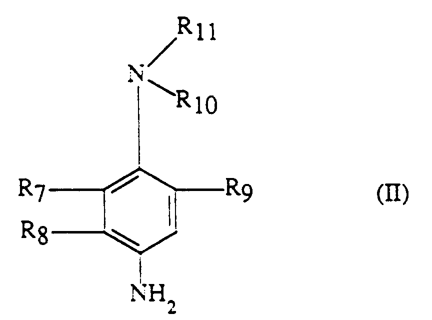 dans laquelle :
R₇, R₈ et R₉, identiques ou différents, représentent un atome d'hydrogène ou d'halogène, un radical alkyle ayant de 1 à 4 atomes de carbone, un radical alcoxy ayant de 1 à 4 atomes de carbone;
R₁₀ et R₁₁, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle; ces groupes alkyle ou alcoxy ayant de 1 à 4 atomes de carbone, ou bien R₁₀ et R₁₁ forment, conjointement avec l'atome d'azote auquel ils sont liés, un hétérocycle pipéridino ou morpholino, sous réserve que R₇ ou R₉ représente un atome d'hydrogène lorsque R₁₀ et R₁₁ ne représentent pas un atome d'hydrogène, ainsi que les sels de ces composés à l'exclusion de la 2,6-diméthylparaphénylènediamine et de la 2,3-diméthylparaphénylènediamine.

Dans les formules (I) et (II) ci-dessus, à titre de radicaux alkyle, on peut citer : méthyle, éthyle, propyle, isopropyle, butyle et isobutyle; à titre de radicaux monohydroxyalkyle, on peut citer β-hydroxyéthyle, β-hydroxypropyle, γ-hydroxypropyle; à titre de radical polyhydroxyalkyle on peut citer β,γ-dihydroxypropyle.

Parmi les composés préférés répondant à la formule (II), on peut citer l'isopropyl-p-phénylènediamine, la p-phénylènediamine, la 2-méthyl-p-phénylènediamine, la méthoxyparaphénylènediamine, la chloroparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl) aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl) aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)phényl]morpholine, la N-[(4'-amino)phényl]pipéridine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl) 4-aminophénol, le 2-méthoxy 4-aminophénol, le 3-méthoxy 4-aminophénol, le 2,5-diméthyl 4-aminophénol, le 2-méthoxyméthyl 4-aminophénol, le 2-aminométhyl 4-aminophénol, le 2-β-hydroxyéthylaminométhyl 4-aminophénol, le 2-éthoxyméthyl 4-aminophénol, le 2-(β-hydroxyéthoxy)méthyl 4-aminophénol, le N-(2-hydroxy 5-aminobenzyl)2-méthoxyéthylamine, le N-(2-hydroxy 5-aminobenzyl) 1-méthoxyisopropylamine, le N-(2-hydroxy 5-aminobenzyl)3-isopropoxypropylamine, le 2-acétylamino 4-aminophénol, l'acide 5-amino salicylique.

Les précurseurs de colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène et les orthophénylènediamines.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

Le pH de la composition appliquée sur les fibres kératiniques, en particulier les cheveux, a une valeur inférieure à 7 et est compris de préférence entre 3 et 6,9. Ce pH est ajusté par l'utilisation d'agents acidifiants bien connus dans le domaine de la teinture des fibres kératiniques, et en particulier des cheveux humains, tels que des acides minéraux ou organiques comme l'acide chlorhydrique, l'acide phosphorique, les acides carboxyliques comme l'acide tartrique, l'acide citrique ou les acides sulfoniques.

L'alcoxymétaphénylènediamine de formule (I) est présente dans la composition appliquée sur les fibres kératiniques, dans des proportions comprises de préférence entre 0,01 et 3,5% en poids par rapport au poids total de la composition.

Les compositions définies ci-dessus appliquées dans la teinture des fibres kératiniques, peuvent également contenir en plus de l'alcoxymétaphénylènediamine de formule (I), d'autres coupleurs connus en eux-mêmes tels que des métadiphénols, des métaaminophénols, des métaphénylènediamines, des méta-N-acylaminophénols, des métauréidophénols, des métacarbalcoxyaminophénols, l'α-naphtol, les coupleurs indoliques, des coupleurs possédant un groupement méthylène actif, tels que les composés dicétoniques, les pyrazolones, à l'exclusion du 2,4-diamino 1,3-diméthoxybenzène.

Parmi ces coupleurs pouvant être utilisés en plus de l'alcoxymétaphénylènediamine de formule (I), on peut citer le 2,4-dihydroxyphénoxyéthanol, le 2,4-dihydroxyanisole, le métaaminophénol, la résorcine, le monométhyléther de résorcine, la 2-méthylrésorcine, le pyrocatéchol, le 2-méthyl 5-N-(β-hydroxyéthyl)aminophénol, le 2-méthyl 5-N-(β-mésylaminoéthyl)aminophénol, la 6-hydroxybenzomorpholine, la 6-aminobenzomorpholine, la 2,4-diaminophénoxyéthylamine, le 2-méthyl 5-aminophénol, le 2,6-diméthyl 3-aminophénol, le 3,4-méthylènedioxyphénol, la 3,4-méthylènedioxyaniline, le 4-hydroxyindole, le 6-hydroxyindole, le 7-hydroxyindole, le 7-aminoindole et leurs sels.

Ces compositions peuvent également contenir des agents tensio-actifs anioniques, cationiques, non ioniques, amphotères ou leurs mélanges.

Parmi ces agents tensio-actifs, on peut citer les alkylbenzènesulfonates, les alkylnaphtalènesulfonates, les sulfates, les éthersulfates et les sulfonates d'alcools gras, les sels d'ammonium quaternaires tels que le bromure de triméthylcétylammonium, le bromure de cétylpyridinium, les éthanolamides d'acides gras éventuellement oxyéthylénés, les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les compositions tinctoriales sont généralement aqueuses, mais eues peuvent également contenir des solvants organiques pour solubiliser des composés qui ne seraient pas suffisamment solubles dans l'eau. Parmi ces solvants, on peut citer à titre d'exemple, les alcanols inférieurs en C₂-C₄ tels que l'éthanol et l'isopropanol, le glycérol, les glycols ou éthers de glycols, comme le butoxy-2 éthanol, l'éthylèneglycol, le propylèneglycol, le monoéthyléther et le monométhyléther du diéthylèneglycol, ainsi que les alcools aromatiques comme l'alcool benzylique ou le phénoxyéthanol, ou les mélanges de ces solvants.

La composition appliquée sur les cheveux peut également renfermer des agents épaississants choisis en particulier parmi l'alginate de sodium, la gomme arabique, les dérivés de cellulose tels que la méthylcellulose, l'hydroxyéthylcellulose, l'hydroxypropylcellulose, l'hydroxyméthylcellulose, la carboxyméthylcellulose, les polymères d'acide acrylique éventuellement réticulés, la gomme de xanthane. On peut également utiliser des agents épaississants minéraux tels que la bentonite.

La composition peut également renfermer les agents antioxydants choisis en particulier parmi le sulfite de sodium, l'acide thioglycolique, le bisulfite de sodium, l'acide ascorbique et l'hydroquinone, ainsi que d'autres adjuvants cosmétiquement acceptables lorsque la composition est destinée à être utilisée pour la teinture des fibres kératiniques humaines, tels que des agents de pénétration, des agents séquestrants, des conservateurs, des tampons, des parfums, etc...

La composition appliquée sur les cheveux peut se présenter sous des formes diverses, telles que de liquides, de crèmes, de gels ou sous toute autre forme appropriée pour réaliser une teinture capillaire. Elle peut être conditionnée en flacon aérosol en présence d'un agent propulseur.

L'invention a également pour objet la composition prête à l'emploi utilisée dans le procédé défini ci-dessus.

Selon une forme de réalisation préférée, le procédé comporte une étape préliminaire consistant à stocker sous forme séparée, d'une part, la composition contenant dans un milieu approprié pour la teinture, le coupleur de formule (I) et les précurseurs de colorants d'oxydation sous forme d'un composant (A) et, d'autre part, une composition renfermant l'agent oxydant tel que défini ci-dessus sous forme d'un composant (B), et à procéder à leur mélange extemporané avant d'appliquer ce mélange sur les fibres kératiniques, comme indiqué ci-dessus. Le composant (A) ne contient pas d'ion iodure en quantité suffisante pour oxyder le coupleur de formule (I).

La composition appliquée sur les fibres kératiniques résulte d'un mélange de 10 à 90% du composant (A) avec 90 à 10% du composant (B) contenant un agent oxydant.

L'invention a également pour objet un agent de teinture des fibres kératiniques, en particulier des cheveux, essentiellement caractérisé par le fait qu'il comporte au moins deux composants, l'un des composants étant constitué par le composant (A) défini ci-dessus et l'autre étant constitué par le composant (B) également défini ci-dessus, le pH des composants (A) et (B) étant tel qu'après mélange dans des proportions de 90 à 10% pour le composant (A) et de 10 à 90% pour le composant (B), la composition résultante ait un pH inférieur à 7.

Dans cette forme de réalisation, le composant (A) qui renferme au moins un coupleur de formule (I) et un précurseur de colorant d'oxydation, a un pH compris entre 3 et 10,5 et peut être ajusté à la valeur choisie au moyen d'agents alcalinisants habituellement utilisés en teinture des fibres kératiniques, tels que l'ammoniaque, les carbonates alcalins, les alcanolamines tels que les mono-, di- et triéthanolamines ainsi que leurs dérivés ou des agents acidifiants classiques, tels que les acides minéraux ou organiques, comme les acides chlorhydrique, phosphorique, les acides carboxyliques tels que l'acide tartrique ou citrique ou les acides sulfoniques.

Cette composition peut renfermer les différents autres adjuvants mentionnés ci-dessus, notamment des coupleurs différents des coupleurs de formule (I), à l'exclusion du 2,4-diamino 1,3-diméthoxybenzène.

L'ensemble des précurseurs de colorants par oxydation de type para et/ou ortho ainsi que les coupleurs, sont présents dans des proportions comprises de préférence entre 0,3 et 7% en poids par rapport au poids total du composant (A). La concentration en coupleur de formule (I) peut varier entre 0,05 et 3,5% en poids par rapport au poids total du composant (A).

Les agents tensio-actifs sont présents dans le composant (A) dans des proportions de 0,1 à 55% en poids. Lorsque le milieu contient des solvants en plus de l'eau, ceux-ci sont présents dans des proportions comprises entre 0,5 et 40% en poids et en particulier entre 5 et 30% en poids par rapport au poids total du composant (A). Les agents épaississants sont présents de préférence dans des proportions comprises entre 0,1 et 5%, et en particulier entre 0,2 et 3% en poids. Les agents anti-oxydants mentionnés ci-dessus sont de préférence présents dans le composant (A) dans des proportions comprises entre 0,02 et 1,5% en poids par rapport au poids total du composant (A).

Le composant (B) renfermant l'agent oxydant tel que défini ci-dessus, a un pH inférieur à 7. Ce pH peut avoir une valeur minimum de 1 et de préférence il est compris entre 1,5 et 3,5. Ce composant (B) peut être acidifié avec le même type d'agents acidifiants que ceux utilisés pour le composant (A).

Il peut se présenter sous forme de liquide plus ou moins épaissi, de lait ou de gel.

Cet agent de teinture à deux composants peut être conditionné dans un dispositif à plusieurs compartiments ou kit de teinture, ou tout autre système de conditionnement à plusieurs compartiments dont l'un renferme le composant (A) et le second renferme le composant (B); ces dispositifs pouvant être équipés d'un moyen permettant de délivrer sur les cheveux le mélange souhaité, tel que les dispositifs décrits dans le brevet US-A-4 823 985 de la demanderesse.

L'invention a également pour objet l'utilisation comme coupleur d'une alcoxymétaphénylènediamine de formule (I) pour la teinture en milieu acide des fibres kératiniques, en association avec des précurseurs de colorants d'oxydation.

Conformément à l'invention, le procédé de teinture consiste à appliquer sur les cheveux le mélange obtenu, à le laisser poser pendant 3 à 40 minutes, puis à rincer les cheveux et éventuellement effectuer un shampooing.

Il est également possible, conformément à l'invention, d'appliquer séparément une composition contenant le coupleur de formule (I), le précurseur de colorant d'oxydation et l'agent oxydant, de façon à ce que le mélange se formant in-situ au niveau des fibres ait un pH inférieur à 7, comme défini ci-dessus.

Les exemples suivants sont destinés à illustrer l'invention.

### EXEMPLES 1 à 23

On procède à la teinture des cheveux en appliquant sur des cheveux naturels gris à 90% de blancs ou sur des cheveux gris permanentés, un mélange extemporané de la composition colorante (A) et de la composition oxydante (B).

Ce mélange présente le pH indiqué dans le tableau des exemples qui suivent.

On laisse agir ce mélange pendant 30 minutes, puis on rince les cheveux, on effectue un shampooing.

Après séchage, les cheveux sont teints dans la nuance précisée au bas du tableau.

### EXEMPLES

| en g | 1 | 2 | 3 | 5 |
|---|---|---|---|---|
| A) Composition colorante | | | | |
| 2-méthoxyméthyl 4-aminophénol | 0,46 | 0,46 | 0,46 | 0,46 |
| Dichlorhydrate de 2,4-diaminoanisole | 0,633 | | | |
| Dichlorhydrate de 2-N-β-hydroxyéthylamino 4-amino 1,3,5-triméthoxybenzène | | 0,945 | | |
| Dichlorhydrate de 2-N-β-hydroxyéthylamino 4-amino 1-méthoxybenzène | | | 0,765 | |
| Dichlorhydrate de 2,4-diamino 1,5-diméthoxybenzène, 1H₂O | | | | 0,777 |
| Support | X | X | X | X |
| Eau qsp | 100 | 100 | 100 | 100 |

| en g | 1 | 2 | 3 | 4 | 5 |
|---|---|---|---|---|---|
| B) Composition oxydante | | | | | |
| Solution d'eau oxygénée à 20 volumes | | | | | |
| Acide phosphorique qs pH | 1,1 | 1,3 | 1,1 | 1,2 | 1,2 |
| pH = mélange p/p A + B | 6,7 | 6,6 | 6,7 | 6,6 | 6,7 |

| Nuances obtenues : | | | | | |
|---|---|---|---|---|---|
| sur cheveux gris naturels à 90% de blancs | blond clair irisé | | irisé cendré légèrement acajou | | irisé cendré |
| sur cheveux gris permanentés | | rouge violacé intense | | cuivré irisé | |

| en g | 7 | 8 | 9 | 10 |
|---|---|---|---|---|
| A) Composition colorante | | | | |
| 2-méthoxyméthyl 4-aminophénol | 0,46 | 0,46 | 0,46 | 0,46 |
| Dichlorhydrate de 2,4-diamino 5-méthoxyphénoxyéthanol | 0,814 | | | |
| Dichlorhydrate de 2,4-diamino 1,3,5-triméthoxybenzène | | 0,813 | | |
| Dichlorhydrate de 2,4-diamino 1,5-di-β-hydroxyéthoxybenzène | | | 0,904 | |
| Dichlorhydrate de 2,4-diaminophénoxyéthanol | | | | 0,723 |
| Support | X | X | X | X |
| Eau qsp | 100 | 100 | 100 | 100 |

| en g | 6 | 7 | 8 | 9 | 10 |
|---|---|---|---|---|---|
| B) Composition oxydante | | | | | |
| Solution d'eau oxygénée à 20 volumes | | | | | |
| Acide phosphorique qs pH | 1,4 | 1,3 | 1,1 | 1,3 | 1,4 |
| pH = mélange p/p A + B | 6,5 | 6,4 | 6,7 | 6,7 | 6,5 |

| Nuances obtenues : | | | | | |
|---|---|---|---|---|---|
| sur cheveux gris naturels à 90% de blancs | | châtain clair violine | | blond foncé irisé profond | |
| sur ch'eveux gris permanentés | blond irisé | | rouge violacé | | blond doré cuivré |

| en g | 11 | 13 | 14 | 15 | 16 |
|---|---|---|---|---|---|
| A) Composition colorante | | | | | |
| Paraphénylènediamine | 0,324 | | | | |
| 3-méthyl 4-aminophénol | | 0,369 | | | 0,369 |
| Para-aminophénol | | | 0,327 | 0,327 | |
| Dichlorhydrate de 2,4-diamino 1,3,5-triméthoxybenzène | 0,813 | 0,813 | | 0,813 | |
| Dichlorhydrate de 2-N-β-hydroxyéthylamino 4-amino 1,3,5-triméthoxybenzène | | | 0,945 | | 0,945 |
| Support | X | X | X | X | X |
| Eau qsp | 100 | 100 | 100 | 100 | 100 |

| en g | 11 | 12 | 13 | 14 | 15 | 16 | 17 |
|---|---|---|---|---|---|---|---|
| B) Composition oxydante | | | | | | | |
| Solution d'eau oxygénée à 20 volumes | | | | | | | |
| Acide phosphorique qs pH | 1,2 | 1,4 | 1,1 | 1,1 | 1,4 | 1,3 | 1,3 |
| pH = mélange p/p A+B | 6,6 | 6,5 | 6,5 | 6,6 | 6,5 | 6,6 | 6,6 |

| Nuances obtenues : | | | | | | | |
|---|---|---|---|---|---|---|---|
| sur cheveux gris naturel à 90% de blancs | bleu profond | | violine | rose violine | | rose violacé | |
| sur cheveux gris permanentés | | violine | | | rouge violine | | rose |

| en g | 18 | 19 | 20 | 21 | 22 | 23 |
|---|---|---|---|---|---|---|
| A) Composition colorante | | | | | | |
| Paraphénylènediamine | 0,327 | | 0,327 | | 0,327 | |
| Para-aminophénol | | 0,327 | | 0,327 | | 0,327 |
| 2,4-diamino 6-méthyl 1-méthoxybenzène dichlorhydrate | 0,675 | 0,675 | | | | |
| 2,4-diamino 1,6-diméthoxybenzène dichlorhydrate | | | 0,723 | 0,723 | | |
| 2,6-diamino 4-méthyl 1-méthoxybenzène dichlorhydrate | | | | | 0,675 | 0,675 |
| Support | X | X | X | X | X | X |
| Eau qsp | 100 | 100 | 100 | 100 | 100 | 100 |

| en g | 18 | 19 | 20 | 21 | 22 | 23 |
|---|---|---|---|---|---|---|
| B) Composition oxydante | | | | | | |
| Solution d'eau oxygénée à 20 volumes | | | | | | |
| Acide phosphorique qs pH | 1,2 | 1,4 | 1,1 | 1,4 | 1,3 | 1,3 |
| pH = mélange p/p A + B | 5,5 | 6,7 | 6,0 | 6,4 | 6,3 | 6,4 |

| Nuances obtenues : | | | | | | |
|---|---|---|---|---|---|---|
| sur cheveux gris naturels à 90% de blancs | beige rosé | | | | | |
| sur cheveux gris permanentés | | châtain clair | bleu nuit | violine clair | auburn clair | châtain clair doré |

| SUPPORT DE COLORATION | |
|---|---|
| - Alcool oléique polyglycérolé à 2 moles de glycérol | 4,0 g |
| - Alcool oléique polyglycérolé à 4 moles de glycérol à 78% de MA | 5,69 g MA |
| - Acide oléique | 3,0 g |
| - Amine oléique à 2 moles d'oxyde d'éthylène, vendue sous la dénomination ETHOMEEN 0 12 par la Société AKZO | 7,0 g |
| - Laurylaminosuccinamate de diéthylaminopropyle, sel de sodium à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de MA | 0,45 g MA |
| - Acétate d'ammonium | 0,8 g |
| - Antioxydant, séquestrant qs | |

## Revendications

1. Procédé de teinture des fibres kératiniques, en particulier des fibres kératiniques humaines telles que les cheveux, caractérisé par le fait qu'on applique sur ces fibres une composition contenant dans un milieu approprié pour la teinture :
- au moins, comme coupleur, une alcoxymétaphénylènediamine de formule (I) suivante : 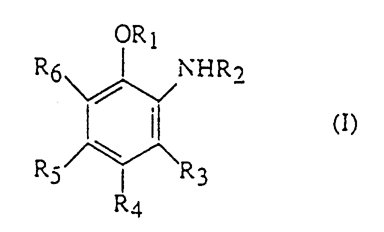 dans laquelle :
R₁ représente un radical alkyle en C₁-C₄, mono- ou polyhydroxyalkyle en C₂-C₄, carboxyalkyle en C₁-C₄ ou aminoalkyle en C₁-C₄ dont le groupe amino peut être mono- ou disubstitué par un alkyle en C₁-C₄;
R₂ représente un atome d'hydrogène, un radical alkyle en C₁-C₄, mono- ou polyhydroxyalkyle en C₂-C₄;
R₃ et R₅, indépendamment l'un de l'autre, représentent H ou OR, R désignant un radical alkyle en C₁-C₄, mono- ou polyhydroxyalkyle en C₂-C₄;
R₄ représente un atome d'hydrogène, un radical alkyle en C₁-C₄ ou NHR';
R₆ représente un atome d'hydrogène, un radical alkyle en C₁-C₄, OR ou NHR', R ayant la signification indiquée ci-dessus;
R' désigne un atome d'hydrogène, un radical alkyle en C₁-C₄, sous réserve que :
a) un et un seul des radicaux R₄ et R₆ désigne NHR';
b) R₃ et OR₁ ne peuvent désigner simultanément méthoxy lorsque R₂, R₅ et R₆ désignent simultanément l'hydrogène et R₄ le groupe NH₂;
c) au moins un des radicaux R₃, R₄, R₅ ou R₆ désigne H;
d) R₃, R₄ et R₅ ne peuvent désigner simultanément l'hydrogène lorsque R₆ désigne NH₂, R₂ désigne l'hydrogène et R₁ désigne méthyle;
e) lorsque R₁ désigne éthyle et R₂, R₃, R₅ et R₆ désignent hydrogène, R₄ ne peut désigner NH₂;
f) R₄ doit représenter NHR' lorsque R₁ désigne carboxyalkyle ou aminoalkyle;
ainsi que les sels de ces composés;
- au moins un précurseur de colorant d'oxydation ou base d'oxydation;
- au moins un agent oxydant;
- le pH de la composition appliquée sur les fibres étant inférieur à 7.

2. Procédé selon la revendication 1, caractérisé par le fait que le coupleur de formule (I) est choisi parmi :
- le 2,4-diaminoanisole,
- le 2-N-β-hydroxyéthylamino 4-amino 1,3,5-triméthoxybenzène,
- le 2-N-β-hydroxyéthylamino 4-amino 1-méthoxybenzène,
- le 2,4-diamino 1,5-diméthoxybenzène,
- le 2,4-diamino 5-méthoxyphénoxyéthanol,
- le 2,4-diamino 1,3,5-triméthoxybenzène,
- le 2,4-diamino 1,5-di-(β-hydroxyéthoxy)benzène,
- le 2,4-diaminophénoxyéthanol,
- le 2,4-diamino 6-méthyl 1-méthoxybenzène,
- le 2,4-diamino 1,6-diméthoxybenzène,
- le 2,6-diamino 4-méthyl 1-méthoxybenzène,
ainsi que leurs sels.

3. Procédé selon la revendication 1 ou 2, caractérisé par le fait que les précurseurs de colorants d'oxydation sont choisis parmi les paraphénylènediamines, les para-aminophénols, les précurseurs hétérocycliques para.

4. Procédé selon la revendication 3, caractérise par le fait que les paraphénylènediamines sont choisies parmi les composés répondant à la formule : 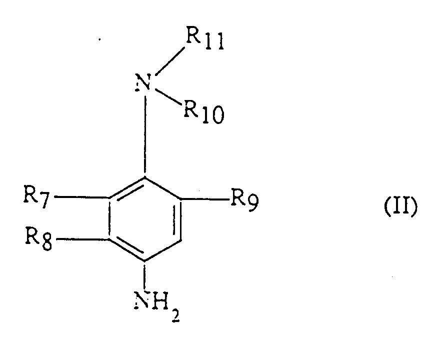 dans laquelle :
R₇, R₈ et R₉, identiques ou différents, représentent un atome d'hydrogène ou d'halogène, un radical alkyle ayant de 1 à 4 atomes de carbone, un radical alcoxy ayant de 1 à 4 atomes de carbone;
R₁₀ et R₁₁, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle; ces groupes alkyle ou alcoxy ayant de 1 à 4 atomes de carbone, ou bien R₁₀ et R₁₁ forment, conjointement avec l'atome d'azote auquel ils sont liés, un hétérocycle pipéridino ou morpholino, sous réserve que R₇ ou R₉ représente un atome d'hydrogène lorsque R₁₀ et R₁₁ ne représentent pas un atome d'hydrogène, ainsi que les sels de ces composés à l'exclusion de la 2,6-diméthylparaphénylènediamine et de la 2,3-diméthylparaphénylènediamine.

5. Procédé selon la revendication 3 ou 4, caractérisé par le fait que les composés de formule (II) sont choisis parmi l'isopropyl-p-phénylènediamine, la p-phénylènediamine, la 2-méthyl-p-phénylènediamine, la méthoxyparaphénylènediamine, la chloroparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl) aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl) aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)phényl]morpholine, la N-[(4'-amino)phényl]pipéridine, sous forme de base libre ou de sels.

6. Procédé selon les revendications 1 à 3, caractérisé par le fait que les p-aminophénols sont choisis parmi le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl) 4-aminophénol, le 2-méthoxy 4-aminophénol, le 3-méthoxy 4-aminophénol, le 2,5-diméthyl 4-aminophénol, le 2-méthoxyméthyl 4-aminophénol, le 2-aminométhyl 4-aminophénol, le 2-β-hydroxyéthylaminométhyl 4-aminophénol, le 2-éthoxyméthyl 4-aminophénol, le 2-(β-hydroxyéthoxy)méthyl 4-aminophénol, le N-(2-hydroxy 5-aminobenzyl)2-méthoxyéthylamine, le N-(2-hydroxy 5-aminobenzyl) 1-méthoxyisopropylamine, le N-(2-hydroxy 5-aminobenzyl)3-iso-propoxypropylamine, le 2-acétylamino 4-aminophénol, l'acide 5-amino salicylique.

7. Procédé selon la revendication 1, caractérisé par le fait que les précurseurs de colorants d'oxydation sont des précurseurs de colorants d'oxydation de type ortho choisis parmi les ortho-aminophénols et les orthophénylènediamines.

8. Procédé selon l'une quelconque des revendications 1 à 7, caractérisé par le fait que l'agent oxydant est choisi parmi le peroxyde d'hydrogène, le peroxyde d'urée, les bromates de métaux alcalins, les persels.

9. Procédé selon l'une quelconque des revendications 1 à 8, caractérisé par le fait que le pH de la composition appliquée sur les fibres kératiniques est compris entre 3 et 6,9.

10. Procédé selon l'une quelconque des revendications 1 à 9, caractérisé par le fait que la composition utilisée pour la teinture des fibres kératiniques contient en plus du coupleur de formule (I), d'autres coupleurs choisis parmi les métadiphénols, les métaaminophénols, les métaphénylènediamines, les méta-N-acylaminophénols, les métauréidophénols, les métacarbalcoxyaminophénols, l'α-naphtol, les coupleurs indoliques, les coupleurs possédant un groupement méthylène actif choisis parmi les composés dicétoniques et les pyrazolones, à l'exclusion du 2,4- diamino 1,3-diméthoxybenzène.

11. Procédé selon la revendication 10, caractérisé par le fait que les coupleurs sont choisis parmi le 2,4-dihydroxyphénoxyéthanol, le 2,4-dihydroxyanisole, le métaaminophénol, la résorcine, le monométhyléther de résorcine, la 2-méthylrésorcine, le pyrocatéchol, le 2-méthyl 5-N-(β-hydroxyéthyl)aminophénol, le 2-méthyl 5-N-(β-mésylaminoéthyl)aminophénol, la 6-hydroxybenzomorpholine, la 6-aminobenzomorpholine, le 2-méthyl 5-aminophénol, le 2,6-diméthyl 3-aminophénol, le 3,4-méthylènedioxyphénol, la 3,4-méthylènedioxyaniline, le 4-hydroxyindole, le 6-hydroxyindole, le 7-hydroxyindole, le 7-aminoindole et leurs sels.

12. Procédé selon l'une quelconque des revendications 1 à 11, caractérisé par le fait que la composition contient des agents tensioactifs anioniques, cationiques, non ioniques, amphotères ou leurs mélanges; des agents épaississants, des agents anti-oxydants et/ou tout autre adjuvant cosmétiquement acceptable.

13. Procédé selon l'une quelconque des revendications 1 à 12, caractérisé par le fait que le milieu approprié pour la teinture est constitué par de l'eau ou un mélange d'eau et d'un solvant choisi parmi les alcanols inférieurs en C₂-C₄, le glycérol, les glycols ou éthers de glycols, le monoéthyléther et le monométhyléther du diéthylèneglycol, les alcools aromatiques ou leurs mélanges.

14. Agent de teinture des fibres kératiniques et en particulier des cheveux, caractérisé par le fait qu'il comporte au moins deux composants; un composant (A) constitué par une composition contenant dans un milieu approprié pour la teinture un coupleur de formule (I) selon la revendication 1 ou 2, et un précurseur de colorant d'oxydation tel que défini dans l'une quelconque des revendications 3 à 7, un composant (B) constitué par une composition contenant dans un milieu approprié pour la teinture, un agent oxydant, le pH des composants (A) et (B) étant tel qu'après mélange dans des proportions de 90 à 10% pour le composant (A), de 10 à 90% pour le composant (B), la composition résultante ait un pH inférieur à 7.

15. Agent selon la revendication 14, caractérisé par le fait que le composant (A) a un pH compris entre 3 et 10,5.

16. Agent selon la revendication 14 ou 15, caractérisé par le fait que le composant (A) contient les précurseurs de colorants par oxydation du type para et/ou ortho ainsi que les coupleurs tels que définis dans les revendications 1, 2 et 10, dans des proportions comprises entre 0,3 et 7% en poids par rapport au poids total du composant (A).

17. Agent selon l'une quelconque des revendications 14 à 16, caractérise par le fait que la concentration en coupleur de formule (I) est comprise entre 0,05 et 3,5% en poids par rapport au poids total du composant (A).

18. Agent selon l'une quelconque des revendications 14 à 17, caractérisé par le fait que le composant (A) contient des agents tensioactifs dans des proportions de 0,1 à 55% en poids, des agents solvants en plus de l'eau dans des proportions comprises entre 0,5 et 40% en poids, des agents épaississants dans des proportions comprises entre 0,1 et 5% en poids, des agents antioxydants dans des proportions comprises entre 0,02 et 1,5% en poids, et/ou tout autre adjuvant cosmétiquement acceptable.

19. Agent selon l'une quelconque des revendications 14 à 18, caractérisé par le fait que le composant (B) a un pH qui a une valeur minimum de 1 et inférieure à 7.

20. Procédé de teinture des fibres kératiniques et en particulier des cheveux, caractérisé par le fait qu'il comporte une première étape consistant à stocker sous forme séparée les composants de l'agent de teinture tel que défini dans l'une quelconque des revendications 14 à 19 et à procéder avant application au mélange des composants (A) et (B) dans des proportions de 10 à 90% pour le composant (A) et de 90 à 10% pour le composant (B), de façon à obtenir une composition ayant un pH inférieur à 7 et d'appliquer ce mélange immédiatement après préparation sur les fibres kératiniques.

21. Dispositif à plusieurs compartiments ou kit de teinture, caractérisé par le fait qu'il comporte au moins deux compartiments dont un premier compartiment renferme le composant (A) tel que défini dans l'une quelconque des revendications 14 à 18, et le second compartiment renferme le composant (B) tel que défini dans les revendications 14 et 19.

22. Dispositif selon la revendication 21, caractérisé par le fait qu'il est équipé d'un moyen permettant de délivrer sur les cheveux le mélange souhaité des composants (A) et (B).

23. Procédé de teinture selon l'une quelconque des revendications 1 à 13, caractérisé par le fait que l'on applique sur les cheveux la composition et qu'on la laisse poser pendant 3 à 40 minutes, que l'on rince les cheveux et qu'on procède éventuellement à un shampooing avant un nouveau rinçage et séchage.

24. Utilisation comme coupleurs des composés de formule (I) selon la revendication 1 ou 2, pour la teinture en milieu acide des fibres kératiniques, en association avec des précurseurs de colorants d'oxydation.

25. Composition de teinture de fibres kératiniques, prête à l'emploi telle que mise en oeuvre dans le procédé selon l'une quelconque des revendications 1 à 13.

26. Composition selon la revendication 25, contenant le coupleur de formule (I) dans des proportions de 0,01 à 3,5% en poids par rapport au poids total de la composition.

## Claims

1. Process for dyeing keratinous fibres, in particular human keratinous fibres such as hair, characterized in that there is applied to these fibres a composition containing, in a medium suitable for dyeing:
- at least, as coupler, one alkoxymetaphenylenediamine of formula (I) below: 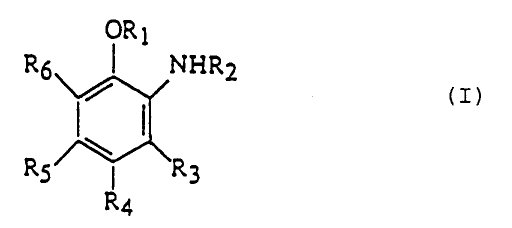 in which:
R₁ represents a C₁-C₄-alkyl radical, a C₂-C₄ mono- or polyhydroxyalkyl radical, a C₁-C₄-carboxyalkyl radical or a C₁-C₄-aminoalkyl radical whose amino group may be mono- or disubstituted by a C₁-C₄-alkyl;
R₂ represents a hydrogen atom, a C₁-C₄-alkyl radical, a C₂-C₄ mono- or polyhydroxyalkyl radical;
R₃ and R₅, independently of each other, represent H or OR, R denoting a C₁-C₄-alkyl radical or a C₂-C₄ mono- or polyhydroxyalkyl radical;
R₄ represents a hydrogen atom, a C₁-C₄-alkyl radical or NHR';
R₆ represents a hydrogen atom, a C₁-C₄-alkyl radical, OR or NHR', R having the meaning given above;
R' denotes a hydrogen atom or a C₁-C₄-alkyl radical; provided that:
a) one and only one of the radicals R₄ and R₆ denotes NHR';
b) R₃ and OR₁ cannot simultaneously denote methoxy when R₂, R₅ and R₆ simultaneously denote hydrogen and R₄ the NH₂ group;
c) at least one of the radicals R₃, R₄, R₅ or R₆ denotes H;
d) R₃, R₄ and R₅ cannot simultaneously denote hydrogen when R₆ denotes NH₂, R₂ denotes hydrogen and R₁ denotes methyl;
e) when R₁ denotes ethyl and R₂, R₃, R₅ and R₆ denote hydrogen, R₄ cannot denote NH₂;
f) R₄ must represent NHR' when R₁ denotes carboxyalkyl or aminoalkyl;
as well as the salts of these compounds;
- at least one oxidation dye precursor or oxidation base;
- at least one oxidising agent;
- the pH of the composition applied to the fibres being less than 7.

2. Process according to Claim 1, characterized in that the coupler of formula (I) is chosen from:
- 2,4-diaminoanisole,
- 2-N-β-hydroxyethylamino-4-amino-1,3,5-trimethoxy benzene,
- 2-N-β-hydroxyethylamino-4-amino-1-methoxybenzene,
- 2,4-diamino-1,5-dimethoxybenzene,
- 2,4-diamino-5-methoxyphenoxyethanol,
- 2,4-diamino-1,3,5-trimethoxybenzene,
- 2,4-diamino-1,5-di-(β-hydroxyethoxy)benzene,
- 2,4-diaminophenoxyethanol,
- 2,4-diamino-6-methyl-1-methoxybenzene,
- 2,4-diamino-1,6-dimethoxybenzene,
- 2,6-diamino-4-methyl-1-methoxybenzene,
as well as their salts.

3. Process according to Claim 1 or 2, characterized in that the oxidation dye precursors are chosen from para-phenylenediamines, para-aminophenols and para heterocyclic precursors.

4. Process according to Claim 3, characterized in that the para-phenylenediamines are chosen from the compounds of formula: 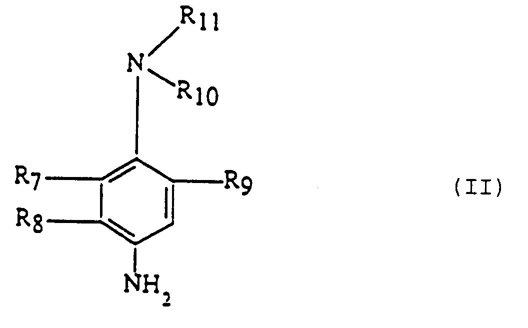 in which:
R₇, R₈ and R₉, which are identical or different, represent a hydrogen or halogen atom, an alkyl radical having 1 to 4 carbon atoms, an alkoxy radical having 1 to 4 carbon atoms;
R₁₀ and R₁₁, which are identical or different, represent a hydrogen atom, an alkyl, hydroxyalkyl, alkoxyalkyl, carbamylalkyl, mesylaminoalkyl, acetylaminoalkyl, ureidoalkyl, carbalkoxyaminoalkyl, sulphoalkyl, piperidinoalkyl or morpholinoalkyl radical; these alkyl or alkoxy groups having 1 to 4 carbon atoms, or alternatively R₁₀ and R₁₁ form, together with the nitrogen atom to which they are attached, a piperidino or morpholino heterocycle, provided that R₇ or R₉ represents a hydrogen atom when R₁₀ and R₁₁ do not represent a hydrogen atom, as well as the salts of these compounds excluding 2,6-dimethylparaphenylenediamine and 2,3-dimethylparaphenylenediamine.

5. Process according to Claim 3 or 4, characterized in that the compounds of formula (II) are chosen from isopropyl-p-phenylenediamine, p-phenylenediamine, 2-methyl-p-phenylenediamine, methoxyparaphenylenediamine, chloroparaphenylenediamine, 2-methyl-5-methoxyparaphenylenediamine, 2,6-dimethyl-5-methoxyparaphenylenediamine, N,N-dimethylparaphenylenediamine, 3-methyl-4-amino-N,N-diethylaniline, N,N-di-(β-hydroxyethyl)paraphenylenediamine, 3-methyl-4-amino-N,N-di-(β-hydroxyethyl)aniline, 3-chloro-4-amino-N,N-di-(β-hydroxyethyl)aniline, 4-amino-N,N-(ethylcarbamylmethyl)aniline, 3-methyl-4-amino-N,N-(ethylcarbamylmethyl)aniline, 4-amino-N,N-(ethyl-β-piperidinoethyl)aniline, 3-methyl-4-amino-N,N-(ethyl-β-piperidinoethyl)aniline, 4-amino-N,N-(ethyl-β-morpholinoethyl)aniline, 3-methyl-4-amino-N,N-(ethyl-β-morpholinoethyl)aniline, 4-amino-N,N-(ethyl-β-acetylaminoethyl)aniline, 4-amino-N-(β-methoxyethyl)aniline, 3-methyl-4-amino-N,N-(ethyl-β-acetylaminoethyl)aniline, 4-amino-N,N-(ethyl-β-mesylaminoethyl)aniline, 3-methyl-4-amino-N,N-(ethyl-β-mesylaminoethyl)aniline, 4-amino-N,N-(ethyl-β-sulphoethyl)aniline, 3-methyl-4-amino-N,N-(ethyl-β-sulphoethyl)aniline, N-[(4'-amino)phenyl]morpholine and N-[(4'-amino)phenyl]piperidine, in the form of the free base or salts.

6. Process according to Claims 1 to 3, characterized in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol, 2-methoxymethyl-4-aminophenol, 2-amino-methyl-4-aminophenol, 2-β-hydroxyethylaminomethyl-4-aminophenol, 2-ethoxymethyl-4-aminophenol, 2-(β-hydroxyethoxy)methyl-4-aminophenol, N-(2-hydroxy-5-aminobenzyl)-2-methoxyethylamine, N-(2-hydroxy-5-aminobenzyl)-1-methoxyisopropylamine, N-(2-hydroxy-5-aminobenzyl)-3-isopropoxypropylamine, 2-acetylamino-4-aminophenol or 5-aminosalicylic acid.

7. Process according to Claim 1, characterized in that the oxidation dye precursors are ortho type oxidation dye precursors chosen from ortho-aminophenols and ortho-phenylenediamines.

8. Process according to any one of Claims 1 to 7, characterized in that the oxidising agent is chosen from hydrogen peroxide, urea peroxide, alkali metal bromates and persalts.

9. Process according to any one of Claims 1 to 8, characterized in that the pH of the composition applied to the keratinous fibres is between 3 and 6.9.

10. Process according to any one of Claims 1 to 9, characterized in that the composition used for dyeing keratinous fibres contains, in addition to the coupler of formula (I), other couplers chosen from meta-diphenols, meta-aminophenols, meta-phenylenediamines, meta-N-acylaminophenols, meta-ureidophenols, meta-carbalkoxyaminophenols, α-naphthol, indole couplers, couplers possessing an active methylene group chosen from diketonic compounds and pyrazolones, excluding 2,4-diamino-1,3-dimethoxybenzene.

11. Process according to Claim 10, characterized in that the couplers are chosen from 2,4-dihydroxyphenoxyethanol, 2,4-dihydroxyanisole, meta-aminophenol, resorcinol, resorcinol monomethyl ether, 2-methylresorcinol, pyrocatechol, 2-methyl-5-N-(β-hydroxyethyl)aminophenol, 2-methyl-5-N-(β-mesylaminoethyl)aminophenol, 6-hydroxybenzomorpholine, 6-aminobenzomorpholine, 2-methyl-5-aminophenol, 2,6-dimethyl-3-aminophenol, 3,4-methylenedioxyphenol, 3,4-methylenedioxyaniline, 4-hydroxyindole, 6-hydroxyindole, 7-hydroxyindole, 7-aminoindole and their salts.

12. Process according to any one of Claims 1 to 11, characterized in that the composition contains anionic, cationic, nonionic or amphoteric surface-active agents or mixtures thereof; thickening agents, antioxidants and/or any other cosmetically acceptable adjuvant.

13. Process according to any one of Claims 1 to 12, characterized in that the medium suitable for dyeing consists of water or a mixture of water and a solvent chosen from C₂-C₄ lower alkanols, glycerol, glycols or glycol ethers, diethylene glycol monoethyl ether and monomethyl ether, aromatic alcohols or mixtures thereof.

14. Agent for dyeing keratinous fibres and in particular hair, characterized in that it comprises at least two components; one component (A) consisting of a composition containing, in a medium suitable for dyeing, a coupler of formula (I) according to Claim 1 or 2, and an oxidation dye precursor as defined in any one of Claims 3 to 7, a component (B) consisting of a composition containing, in a medium suitable for dyeing, an oxidising agent, the pH of the components (A) and (B) being such that after mixing in proportions of 90 to 10% for the component (A), of 10 to 90% for the component (B), the resulting composition has a pH of less than 7.

15. Agent according to Claim 14, characterized in that the component (A) has a pH of between 3 and 10.5.

16. Agent according to Claim 14 or 15, characterized in that the component (A) contains para and/or ortho type oxidation dye precursors as well as couplers as defined in Claims 1, 2 and 10, in proportions of between 0.3 and 7% by weight relative to the total weight of the component (A).

17. Agent according to any one of Claims 14 to 16, characterized in that the concentration of the coupler of formula (I) is between 0.05 and 3.5% by weight relative to the total weight of the component (A).

18. Agent according to any one of Claims 14 to 17, characterized in that the component (A) contains surface-active agents in proportions of 0.1 to 55% by weight, solvent agents, in addition to water, in proportions of between 0.5 and 40% by weight, thickening agents in proportions of between 0.1 and 5% by weight, antioxidants in proportions of between 0.02 and 1.5% by weight, and/or any other cosmetically acceptable adjuvant.

19. Agent according to any one of Claims 14 to 18, characterized in that the component (B) has a pH which has a minimum value of 1 and a value of less than 7.

20. Process for dyeing keratinous fibres, and in particular hair, characterized in that it comprises a first stage consisting in storing, in separate form, the components of the dyeing agent as defined in any one of Claims 14 to 19, and in mixing, before application, the components (A) and (B) in proportions of 10 to 90% for the component (A), and of 90 to 10% for the component (B), so as to obtain a composition having a pH of less than 7 and in applying this mixture to the keratinous fibres immediately after preparation.

21. Multi-compartment device or dyeing kit, characterized in that it comprises at least two compartments, of which a first compartment contains the component (A) as defined in any one of Claims 14 to 18, and the second compartment contains the component (B) as defined in Claims 14 and 19.

22. Device according to Claim 21, characterized in that it is equipped with means which make it possible to apply to the hair the desired mixture of the components (A) and (B).

23. Dyeing process according to any one of Claims 1 to 13, characterized in that the composition is applied to the hair and in that it is allowed to act for 3 to 40 minutes, in that the hair is rinsed and in that it is optionally shampooed before another rinsing and drying.

24. Use, as couplers, of the compounds of formula (I) according to Claim 1 or 2, for dyeing keratinous fibres in acidic medium, in combination with oxidation dye precursors.

25. Ready-for-use composition for dyeing keratinous fibres, as implemented in the process according to any one of Claims 1 to 13.

26. Composition according to Claim 25, containing the coupler of formula (I) in proportions of 0.01 to 3.5% by weight relative to the total weight of the composition.

## Patentansprüche

1. Verfahren zum Färben von Keratinfasern und insbesondere von menschlichen Keratinfasern, wie dem Haar, dadurch gekennzeichnet, daß auf die Fasern eine Zusammensetzung aufgetragen wird, die in einem zum Färben geeigneten Medium enthält:
- mindestens ein Alkoxy-m-phenylendiamin der folgenden Formel (I) als Kuppler: 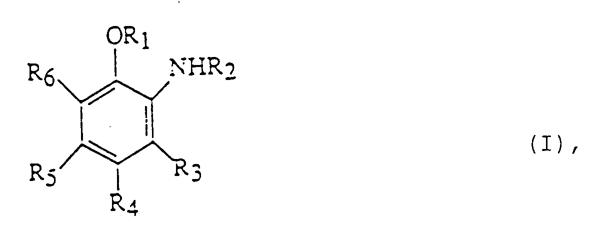 worin bedeuten:
R₁ eine C₁₋₄-Alkylgruppe, eine C₂₋₄-Monohydroxyalkylgruppe, eine C₂₋₄-Polyhydroxyalkylgruppe, eine C₁₋₄-Carboxyalkylgruppe oder eine C₁₋₄-Aminoalkylgruppe, wobei die Aminogruppe ein- oder zweifach mit einer C₁₋₄-Alkylgruppe substituiert sein kann;
R₂ Wasserstoff, eine C₁₋₄-Alkylgruppe, eine C₂₋₄-Monohydroxyalkylgruppe oder eine C₂₋₄-Polyhydroxyalkylgruppe;
R₃ und R₅ unabhängig voneinander H oder OR, wobei R eine C₁₋₄-Alkylgruppe, eine C₂₋₄-Monohydroxyalkylgruppe oder eine C₂₋₄-Polyhydroxyalkylgruppe bedeutet;
R₄ Wasserstoff, eine C₁₋₄-Alkylgruppe oder NHR';
R₆ Wasserstoff, eine C₁₋₄-Alkylgruppe, OR oder NHR', wobei R die oben angegebene Bedeutung aufweist;
wobei R' Wasserstoff oder eine C₁₋₄-Alkylgruppe bedeutet,
mit der Maßgabe, daß
a) nur eine der Gruppen R₄ und R₆ NHR' bedeutet;
b) R₃ und OR₁ nicht gleichzeitig Methoxy bedeuten können, wenn R₂, R₅ und R₆ gleichzeitig Wasserstoff bedeuten und R₄ NH₂ ist;
c) mindestens eine der Gruppen R₃, R₄, R₅ oder R₆ Wasserstoff bedeutet;
d) R₃, R₄ und R₅ nicht gleichzeitig Wasserstoff bedeuten, wenn R₆ NH₂, R₂ Wasserstoff und R₁ Methyl bedeutet;
e) wenn R₁ Ethyl und R₂, R₃, R₅ und R₆ H bedeuten, R₄ nicht NH₂ bedeuten kann;
f) R₄ NHR' bedeuten muß, wenn R₁ Carboxyalkyl oder Aminoalkyl bedeutet;
sowie die Salze dieser Verbindungen;
- mindestens ein Farbstoffvorprodukt eines Oxidationsfarbstoffes oder eine Oxidationsbase;
- mindestens ein Oxidationsmittel;
wobei der pH-Wert der auf die Fasern aufgetragenen Zusammensetzungen unter 7 liegt.

2. Verfahren nach Anspruch 1, dadurch gekennzeichnet, daß der Kuppler der Formel (I) ausgewählt ist unter:
- 2,4-Diaminoanisol,
- 2-N-β-Hydroxyethylamino-4-amino-1,3,5-trimethoxybenzol,
- 2-N-β-Hydroxyethylamino-4-amino-1-methoxybenzol,
- 2,4-Diamino-1,5-dimethoxybenzol,
- 2,4-Diamino-5-methoxyphenoxyethanol,
- 2,4-Diamino-1,3,5-trimethoxybenzol,
- 2,4-Diamino-1,5-di-(β-hydroxyethoxy)benzol,
- 2,4-Diamino-phenoxyethanol,
- 2,4-Diamino-6-methyl-1-methoxybenzol,
- 2,4-Diamino-1,6-dimethoxybenzol,
- 2,6-Diamino-4-methyl-1-methoxybenzol,
sowie deren Salzen.

3. Verfahren nach Anspruch 1 oder 2, dadurch gekennzeichnet, daß die Farbstoffvorprodukte von Oxidationsfarbstoffen unter den p-Phenylendiaminen, p-Aminophenolen und heterocyclischen p-Precursoren ausgewählt sind.

4. Verfahren nach Anspruch 3, dadurch gekennzeichnet, daß die p-Phenylendiamine unter den Verbindungen der folgenden Formel (II) ausgewählt sind: 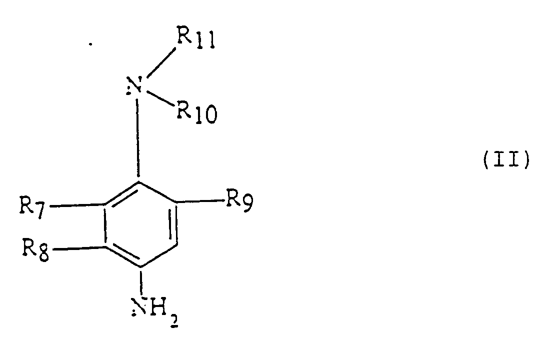 worin bedeuten:
R₇, R₈ und R₉, die identisch oder voneinander verschieden sind, Wasserstoff, Halogen, C₁₋₄-Alkyl oder C₁₋₄-Alkoxy;
R₁₀ und R₁₁, die identisch oder voneinander verschieden sind, Wasserstoff, Alkyl, Hydroxyalkyl, Alkoxyalkyl, Carbamoylalkyl, Mesylaminoalkyl, Acetylaminoalkyl, Ureidoalkyl, Carbalkoxyaminoalkyl, Sulfoalkyl, Piperidinoalkyl und Morpholinoalkyl, wobei die Alkyl- oder Alkoxygruppen 1 bis 4 Kohlenstoffatome aufweisen, oder R₁₀ und R₁₁ bilden mit dem Stickstoffatom, an das sie gebunden sind, einen Piperidino- oder Morpholino-Heterocyclus,
mit der Maßgabe, daß R₇ oder R₉ Wasserstoff bedeutet, wenn R₁₀ und R₁₁ kein Wasserstoffatom bedeuten, sowie die Salze dieser Verbindungen außer 2,6-Dimethyl-p-phenylendiamin und 2,3-Dimethyl-p-phenylendiamin.

5. Verfahren nach Anspruch 3 oder 4, dadurch gekennzeichnet, daß die Verbindungen der Formel (II) ausgewählt sind unter: Isopropyl-p-phenylendiamin, p-Phenylendiamin, 2-Methyl-p-phenylendiamin, Methoxy-p-phenylendiamin, Chlor-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethyl-anilin, N,N-Di-(β-hydroxy-ethyl)-p-phenylendiamin, 3-Methyl-4-amino-N,N-(β-hydroxyethyl)anilin, 3-Chlor-4-amino-N,N-(β-hydroxyethyl)-anilin, 4-Amino-N,N-(ethyl, carbamoylmethyl)-anilin, 3-Methyl-4-amino-N,N-(ethyl, carbamoylmethyl)-anilin, 4-Amino-N,N-(ethyl, β-piperidinoethyl)-anilin, 3-Methyl-4-amino-N,N-(ethyl, β-piperidinoethyl)-anilin, 4-Amino-N,N-(ethyl, β-morpholinoethyl)-anilin, 3-Methyl-4-amino-N,N-(ethyl, β-morpholinoethyl)-anilin, 4-Amino-N,N-(ethyl, β-acetylaminoethyl)-anilin, 4-Amino-N-(β-methoxyethyl)-anilin, 3-Methyl-4-amino-N,N-(ethyl, β-acetylamino-ethyl)-anilin, 4-Amino-N,N-(ethyl, β-mesylaminoethyl)-anilin, 3-Methyl-4-amino-N,N-(ethyl, β-mesylamino-ethyl)-anilin, 4-Amino-N,N-(ethyl, β-sulfoethyl)-anilin, 3-Methyl-4-amino-N,N-(ethyl, β-sulfoethyl)-anilin, N-[(4'-Amino)phenyl]-morpholin und N-[(4'-Amino)phenyl]-piperidin in Form der freien Basen oder in Salzform.

6. Verfahren nach den Ansprüchen 1 bis 3, dadurch gekennzeichnet, daß die p-Aminophenole ausgewählt sind unter: p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-amino-phenol, 3-Methoxy-4-aminophenol, 2,5-Dimethyl-4-aminophenol, 2-Methoxymethyl-4-aminophenol, 2-Aminomethyl-4-aminophenol, 2-β-Hydroxyethylaminomethyl-4-aminophenol, 2-Ethoxymethyl-4-aminophenol, 2-(β-Hydroxyethoxy)methyl-4-aminophenol, N-(2-Hydroxy-5-aminobenzyl)-2-methoxyethylamin, N-(2-Hydroxy-5-aminobenzyl)-1-methoxyisopropylamin, N-(2-Hydroxy-5-aminobenzyl)-3-isopropoxypropylamin, 2-Acetylamino-4-aminophenol und 5-Amino-salicylsäure.

7. Verfahren nach Anspruch 1, dadurch gekennzeichnet, daß die Farbstoffvorprodukte von Oxidationsfarbstoffen Farbstoffvorprodukte von Oxidationsfarbstoffen vom ortho-Typ sind, die unter den o-aminophenolen und o-Phenylendiaminen ausgewählt sind.

8. Verfahren nach einem der Ansprüche 1 bis 7, dadurch gekennzeichnet, daß das Oxidationsmittel unter Wasserstoffperoxid, Harnstoffperoxid, Alkalimetallbromaten und Persäuren ausgewählt ist.

9. Verfahren nach einem der Ansprüche 1 bis 8, dadurch gekennzeichnet, daß der pH-Wert der auf die Keratinfasern aufgetragenen Zusammensetzung im Bereich von 3 bis 6,9 liegt.

10. Verfahren nach einem der Ansprüche 1 bis 9, dadurch gekennzeichnet, daß die zum Färben von Keratinfasern verwendete Zusammensetzung neben dem Kuppler der Formel (I) weitere Kuppler enthält, die unter den m-Dihydroxybenzolen, m-Aminophenolen, m-Phenylendiaminen, m-N-Acylaminophenolen, m-Ureidophenolen, m-Carbalkoxyaminophenolen, α-Naphthol, Indolkupplern und Kupplern, die eine aktive Methylengruppe aufweisen, wie Diketonverbindungen und Pyrazolone, ausgewählt sind, wobei 2,4-Diamino-1,3-dimethoxybenzol ausgenommen ist.

11. Verfahren nach Anspruch 10, dadurch gekennzeichnet, daß die Kuppler unter 2,4-Dihydroxyphenoxyethanol, 2,4-Dihydroxyanisol, m-Aminophenol, Resorcin, Resorcinmonomethylether, 2-Methylresorcin, Pyrocatechin, 2-Methyl-5-N-(β-hydroxyethyl)-aminophenol, 2-Methyl-5-N-(β-mesylaminoethyl)-aminophenol, 6-Hydroxy-benzomorpholin, 6-Amino-benzomorpholin, 2-Methyl-5-aminophenol, 2,6-Dimethyl-3-aminophenol, 3,4-Methylendioxyphenol, 3,4-Methylen-dioxyanilin, 4-Hydroxyindol, 6-Hydroxyindol, 7-Hydroxy-indol, 7-Aminoindol und deren Salzen ausgewählt sind.

12. Verfahren nach einem der Ansprüche 1 bis 11, dadurch gekennzeichnet, daß die Zusammensetzung anionische, kationische, nichtionische oder amphotere grenzflächenaktive Stoffe und deren Gemische, Verdickungsmittel, Antioxidantien und/oder beliebige weitere kosmetisch akzeptable Hilfsstoffe enthält.

13. Verfahren nach einem der Ansprüche 1 bis 12, dadurch gekennzeichnet, daß das zum Färben geeignete Medium aus Wasser oder einem Gemisch von Wasser und einem Lösungsmittel besteht, das unter den niederen Alkanolen mit 2 bis 4 Kohlenstoffatomen, Glycerin, Glykolen oder Glykolethern, Diethylenglykolmonoethylether und Diethylenglykolmonomethylether, aromatischen Alkoholen oder deren Gemischen ausgewählt ist.

14. Mittel zum Färben von Keratinfasern und insbesondere menschlichen Keratinfasern, dadurch gekennzeichnet, daß es zwei Komponenten aufweist: eine Komponente (A), die aus einer Zusammensetzung besteht, die in einem zum Färben geeigneten Medium den Kuppler der Formel (I) nach Anspruch 1 oder 2 und ein Farbstoffvorprodukt eines Oxidationsfarbstoffes nach Anspruch 3 bis 7 enthält, und eine Komponente (B), die aus einer Zusammensetzung besteht, die in einem zum Färben geeigneten Medium das Oxidationsmittel enthält, wobei der pH-Wert der Komponenten (A) und (B) so ist, daß nach dem Mischen von 90 bis 10 % Komponente (A) mit 10 bis 90 % Komponente (A) das resultierende Gemisch einen pH-Wert unter 7 aufweist.

15. Mittel nach Anspruch 14, dadurch gekennzeichnet, daß die Komponente (A) einen pH-Wert im Bereich von 3 bis 10,5 aufweist.

16. Mittel nach Anspruch 14 oder 15, dadurch gekennzeichnet, daß die Komponente (A) die Farbstoffvorprodukte von Oxidationsfarbstoffen vom para- und/oder ortho-Typ sowie Kuppler nach Anspruch 1, 2 und 10 in Mengenanteilen im Bereich von 0,3 bis 7 Gew.-%, bezogen auf das Gesamtgewicht der Komponente (A), enthält.

17. Mittel nach einem der Ansprüche 14 bis 16, dadurch gekennzeichnet, daß die Konzentration des Kupplers der Formel (I) im Bereich von 0,05 bis 3,5 Gew.-%, bezogen auf das Gesamtgewicht der Komponente (A), liegt.

18. Mittel nach einem der Ansprüche 14 bis 17, dadurch gekennzeichnet, daß die Komponente (A) grenzflächenaktive Stoffe in Mengenanteilen von 0,1 bis 55 Gew.-%, neben Wasser Lösungsmittel in Mengenanteilen im Bereich von 0,5 bis 40 Gew.-%, Verdickungsmittel in Mengenanteilen im Bereich von 0,1 bis 5 Gew.-%, Antioxidantien in Mengenanteilen im Bereich von 0,02 bis 1,5 Gew.-% und/oder beliebige weitere kosmetisch akzeptable Hilfsstoffe enthält.

19. Mittel nach einem der Ansprüche 14 bis 18, dadurch gekennzeichnet, daß die Komponente (B) einen pH-Wert von minimal 1 und unter 7 aufweist.

20. Verfahren zum Färben von Keratinfasern und insbesondere von Haaren, dadurch gekennzeichnet, daß es einen ersten Schritt umfaßt, der darin besteht, die Komponenten des Mittels zum Färben nach einem der Ansprüche 14 bis 19 getrennt voneinander aufzubewahren und vor der Anwendung die Komponenten (A) und (B) in Mengenanteilen von 10 bis 90 % Komponente (A) und 90 bis 10 % Komponente (B) so zu vermischen, daß eine Zusammensetzung mit einem pH-Wert unter 7 erhalten wird, und das Gemisch unmittelbar nach der Herstellung auf die Keratinfasern aufzutragen.

21. Vorrichtung mit mehreren Anteilungen oder Kit zum Färben, dadurch gekennzeichnet, daß es mindestens zwei Abteilungen aufweist, wobei eine erste Abteilung die Komponente (A) nach einem der Ansprüche 14 bis 18 und die zweite Abteilung die Komponente (B) nach einem der Ansprüche 14 bis 19 enthält.

22. Vorrichtung nach Anspruch 21, dadurch gekennzeichnet, daß sie mit einem Mittel ausgestattet ist, mit dem das gewünschte Gemisch aus den Komponenten (A) und (B) auf das Haar aufgebracht werden kann.

23. Verfahren zum Färben nach einem der Ansprüche 1 bis 13, dadurch gekennzeichnet, daß die Zusammensetzung auf das Haar aufgetragen und 3 bis 40 min einwirken gelassen wird, worauf das Haar gespült, gegebenenfalls mit Haarwaschmittel gewaschen und nochmals gespült und getrocknet wird.

24. Verwendung der Verbindungen der Formel (I) nach Anspruch 1 oder 2 als Kuppler in Kombination mit Farbstoffvorprodukten von Oxidationsfarbstoffen zum Färben von Keratinfasern in einem sauren Medium.

25. Gebrauchsfertige Zusammensetzung zum Färben von Keratinfasern, die in dem Verfahren nach einem der Ansprüche 1 bis 13 verwendet wird.

26. Zusammensetzung nach Anspruch 25, die den Kuppler der Formel (I) in einem Mengenanteil im Bereich von 0,01 bis 3,5 Gew.-%, bezogen auf das Gesamtgewicht der Zusammensetzung, enthält.
